# EUROPÄISCHE PATENTANMELDUNG

(11) **EP 2 770 064 A1**
(43) Veröffentlichungstag der Anmeldung: **27.08.2014**
(21) Anmeldenummer: 13156363.7
(22) Anmeldetag: 22.02.2013
(51) Int. Cl.: C12Q 1/54, G01N 33/535

(54) **Hocheffiziente Herstellung von Blutglucose-Teststreifen**

(71) Anmelder: F. Hoffmann-La Roche AG, 4070 Basel (CH); Roche Diagnostics GmbH, 68305 Mannheim (DE)
(72) Erfinder: Die Erfindernennung liegt noch nicht vor
(74) Vertreter: Weiss, Wolfgang

(57) **Zusammenfassung**

Die vorliegende Erfindung betrifft ein Verfahren zur Herstellung von diagnostischen Elementen, ein diagnostisches Produkt, welches ein stabiles chemisches Nachweisreagenz umfasst, sowie die Verwendung dieses stabilen chemischen Nachweisreagenzes.

SEQUENCE LISTING

<110>  F. Hoffmann - La Roche AG
       Roche Diagnostics GmbH

<120>  Hocheffiziente Herstellung von Blutglucose-Teststreifen

<130>  46199P EP

<160>  2

<170>  PatentIn version 3.3

<210>  1
<211>  261
<212>  PRT
<213>  Artificial

<220>
<223>  Mutant of glucose dehydrogenase from Bacillus subtilis


<220>
<221>  MUTAGEN
<222>  (96)..(96)

<220>
<221>  MUTAGEN
<222>  (170)..(170)

<400>  1

Met Tyr Pro Asp Leu Lys Gly Lys Val Val Ala Ile Thr Gly Ala Ala
1               5                   10                  15


Ser Gly Leu Gly Lys Ala Met Ala Ile Arg Phe Gly Lys Glu Gln Ala
            20                  25                  30


Lys Val Val Ile Asn Tyr Tyr Ser Asn Lys Gln Asp Pro Asn Glu Val
        35                  40                  45


Lys Glu Glu Val Ile Lys Ala Gly Gly Glu Ala Val Val Val Gln Gly
    50                  55                  60


Asp Val Thr Lys Glu Glu Asp Val Lys Asn Ile Val Gln Thr Ala Ile
65                  70                  75                  80


Lys Glu Phe Gly Thr Leu Asp Ile Met Ile Asn Asn Ala Gly Leu Gly
                85                  90                  95


Asn Pro Val Pro Ser His Glu Met Pro Leu Lys Asp Trp Asp Lys Val
            100                 105                 110


Ile Gly Thr Asn Leu Thr Gly Ala Phe Leu Gly Ser Arg Glu Ala Ile
        115                 120                 125

Lys Tyr Phe Val Glu Asn Asp Ile Lys Gly Asn Val Ile Asn Met Ser
    130                 135                 140

Ser Val His Glu Val Ile Pro Trp Pro Leu Phe Val His Tyr Ala Ala
145                 150                 155                 160

Ser Lys Gly Gly Ile Lys Leu Met Thr Lys Thr Leu Ala Leu Glu Tyr
                165                 170                 175

Ala Pro Lys Gly Ile Arg Val Asn Asn Ile Gly Pro Gly Ala Ile Asn
            180                 185                 190

Thr Pro Ile Asn Ala Glu Lys Phe Ala Asp Pro Lys Gln Lys Ala Asp
        195                 200                 205

Val Glu Ser Met Ile Pro Met Gly Tyr Ile Gly Glu Pro Glu Glu Ile
    210                 215                 220

Ala Ala Val Ala Val Trp Leu Ala Ser Lys Glu Ser Ser Tyr Val Thr
225                 230                 235                 240

Gly Ile Thr Leu Phe Ala Asp Gly Gly Met Thr Lys Tyr Pro Ser Phe
                245                 250                 255

Gln Ala Gly Arg Gly
            260

<210> 2
<211> 261
<212> PRT
<213> Artificial

<220>
<223> Mutant of glucose dehydrogenase from Bacillus subtilis

<220>
<221> MUTAGEN
<222> (170)..(170)

<220>
<221> MUTAGEN
<222> (252)..(252)

<400> 2

Met Tyr Pro Asp Leu Lys Gly Lys Val Val Ala Ile Thr Gly Ala Ala
1               5                   10                  15

Ser Gly Leu Gly Lys Ala Met Ala Ile Arg Phe Gly Lys Glu Gln Ala
            20                  25                  30

Lys Val Val Ile Asn Tyr Tyr Ser Asn Lys Gln Asp Pro Asn Glu Val
        35                  40                  45

Lys Glu Glu Val Ile Lys Ala Gly Gly Glu Ala Val Val Val Gln Gly
    50                  55                  60

Asp Val Thr Lys Glu Glu Asp Val Lys Asn Ile Val Gln Thr Ala Ile
65                  70                  75                  80

Lys Glu Phe Gly Thr Leu Asp Ile Met Ile Asn Asn Ala Gly Leu Glu
                85                  90                  95

Asn Pro Val Pro Ser His Glu Met Pro Leu Lys Asp Trp Asp Lys Val
            100                 105                 110

Ile Gly Thr Asn Leu Thr Gly Ala Phe Leu Gly Ser Arg Glu Ala Ile
        115                 120                 125

Lys Tyr Phe Val Glu Asn Asp Ile Lys Gly Asn Val Ile Asn Met Ser
    130                 135                 140

Ser Val His Glu Val Ile Pro Trp Pro Leu Phe Val His Tyr Ala Ala
145                 150                 155                 160

Ser Lys Gly Gly Ile Lys Leu Met Thr Lys Thr Leu Ala Leu Glu Tyr
                165                 170                 175

Ala Pro Lys Gly Ile Arg Val Asn Asn Ile Gly Pro Gly Ala Ile Asn
            180                 185                 190

Thr Pro Ile Asn Ala Glu Lys Phe Ala Asp Pro Lys Gln Lys Ala Asp
        195                 200                 205

Val Glu Ser Met Ile Pro Met Gly Tyr Ile Gly Glu Pro Glu Glu Ile
    210                 215                 220

Ala Ala Val Ala Val Trp Leu Ala Ser Lys Glu Ser Ser Tyr Val Thr
225                 230                 235                 240

Gly Ile Thr Leu Phe Ala Asp Gly Gly Met Thr Leu Tyr Pro Ser Phe
                245                 250                 255

Gln Ala Gly Arg Gly
            260

## Beschreibung

Die vorliegende Erfindung betrifft ein Verfahren zur Herstellung von diagnostischen Elementen, ein diagnostisches Produkt, welches ein stabiles chemisches Nachweisreagenz umfasst, sowie die Verwendung dieses stabilen chemischen Nachweisreagenzes.

Diagnostische Elemente sind wichtige Bestandteile klinisch relevanter Analyseverfahren. Hierbei steht die Messung von Analyten, z.B. Metaboliten oder Substraten, im Vordergrund, welche beispielsweise mit Hilfe eines für den Analyten spezifischen Enzyms direkt oder indirekt bestimmt werden. Die Analyten werden hierbei mit Hilfe eines Enzym-Coenzym-Komplexes umgesetzt und unter Verwendung geeigneter Mittel anschließend quantifiziert.

Dabei wird der zu bestimmende Analyt mit einem geeigneten Enzym und einem Coenzym in Kontakt gebracht, wobei das Enzym meist in katalytischen Mengen eingesetzt wird. Das Coenzym wird durch die enzymatische Reaktion physikochemisch verändert, z.B. oxidiert bzw. reduziert, und der Vorgang beispielsweise elektrochemisch oder photometrisch erfasst. Eine Kalibrierung liefert einen direkten Zusammenhang zwischen dem Messwert und der Konzentration des zu bestimmenden Analyten.

Aus dem Stand der Technik bekannte diagnostische Elemente zeichnen sich durch eine zeitlich begrenzte Haltbarkeit sowie durch spezielle Anforderungen an die Umgebung, wie Kühlung oder trockene Lagerung, zur Erzielung dieser Haltbarkeit aus. Bei bestimmten Anwendungsformen, z.B. bei Tests, die vom Endverbraucher selbst durchgeführt werden, wie etwa beim Blutglucose-Selbstmonitoring, können daher durch eine falsche, unbemerkte Fehllagerung des Messsystems Fehlergebnisse vorkommen, welche vom Verbraucher kaum zu erkennen sind und ggf. zu einer Fehlbehandlung der jeweiligen Erkrankung führen können.

Die Fehlergebnisse beruhen in erster Linie auf der Tatsache, dass die in derartigen diagnostischen Elementen eingesetzten Substanzen, insbesondere Enzyme, Coenzyme oder/und Mediatoren, im Allgemeinen empfindlich auf Feuchtigkeit, Wärme oder/und Licht reagieren und mit der Zeit inaktiviert werden. Dies hat u.a. zur Folge, dass während des Herstellungsprozesses bestimmte Standzeiten einer in wässriger Lösung angesetzten Testchemie nicht überschritten werden dürfen und die auf einen geeigneten Träger aufgebrachte Testchemie vor dem nächsten Verarbeitungsschritt nicht übermäßig lange aufbewahrt werden kann.

Um diesen durch die Testchemie vorgegebenen Beschränkungen Rechnung zu tragen, ist die Größe der Chargen einer für die Herstellung diagnostischer Elemente verwendeten Testchemie üblicherweise begrenzt. Auf diese Weise kann sichergestellt werden, dass eine einzelne Charge der Testchemie im wesentlichen homogen ist und die in der Testchemie enthaltenen Substanzen darüber hinaus auch nach ihrer Verarbeitung zu einem diagnostischen Element noch eine ausreichend hohe Aktivität für die nachfolgende Detektion eines zu bestimmenden Analyten besitzen.

Da jede Charge einer zur Herstellung diagnostischer Elemente verwendeten Testchemie aus Qualitätsgründen üblicherweise eine eigene Chargenkodierung erhält, ergibt sich indessen das Problem, dass eine einzelne Chargenkodierung lediglich eine geringe Menge an diagnostischen Elementen erfasst. Um eine große Anzahl an diagnostischen Elementen zu kodieren, muss folglich eine Vielzahl unterschiedlicher Chargenkodierungen erstellt werden, was mit einem erheblichen zeitlichen, technischen und finanziellen Mehraufwand verbunden ist.

Die der Erfindung zugrunde liegende Aufgabe bestand somit darin, ein Verfahren zur Herstellung diagnostischer Elemente bereitzustellen, bei welchem die Nachteile des Standes der Technik zumindest teilweise beseitigt sind. Insbesondere sollte das Verfahren die Bereitstellung großer und homogener Chargen einer zu verwendenden Testchemie ermöglichen, eine hohe Homogenität nacheinander produzierter Chargen diagnostischer Elemente gewährleisten, die Verwendung einer einzigen Chargenkodierung für eine große Anzahl an diagnostischen Elementen gestatten, und darüber hinaus die Möglichkeit der Kodierung einzelner diagnostischer Elemente bieten.

Diese Aufgabe wurde erfindungsgemäß gelöst mittels eines Verfahrens zur Herstellung von diagnostischen Elementen, umfassend die Schritte:
(a) Bereitstellen einer Charge eines chemischen Nachweisreagenzes, umfassend ein Coenzym-abhängiges Enzym und ein artifizielles Coenzym,
(b) Beschichten eines ersten Trägers mit einem ersten Teil der Charge des chemischen Nachweisreagenzes,
(c) Vereinzeln des beschichteten ersten Trägers zu mehreren ersten diagnostischen Elementen,
(d) Erstellen einer Chargenkodierung für die Charge des chemischen Nachweisreagenzes anhand mindestens eines der ersten diagnostischen Elemente,
(e) Beschichten eines zweiten Trägers mit einem zweiten Teil der Charge des chemischen Nachweisreagenzes,
(f) Vereinzeln des beschichteten zweiten Trägers zu mehreren zweiten diagnostischen Elementen, und
(g) ggf. Überprüfen und Verpacken der zweiten diagnostischen Elemente,
**dadurch gekennzeichnet,**
dass die zweiten diagnostischen Elemente vor dem Vereinzeln des beschichteten zweiten Trägers mit der in Schritt (d) erstellten Chargenkodierung versehen werden.

Überraschenderweise wurde im Rahmen der vorliegenden Erfindung festgestellt, dass ein chemisches Nachweisreagenz, welches eine Kombination aus einem Coenzym-abhängigen Enzym und einem artifiziellen Coenzym umfasst, aufgrund seiner hohen Stabilität gegenüber Feuchtigkeit, Wärme und Licht die Herstellung, Überprüfung, Verpackung und Handhabung diagnostischer Elemente erheblich vereinfacht. Weiterhin ergeben sich durch den Einsatz eine solchen chemischen Nachweisreagenzes zeitliche und finanzielle Vorteile, welche speziell bei der großindustriellen Produktion von diagnostischen Testelement eine überragende Bedeutung besitzen.

Der erste Schritt des erfindungsgemäßen Verfahrens erfordert die Bereitstellung einer Charge eines chemischen Nachweisreagenzes, welches u.a. ein Coenzym-abhängiges Enzym, wie beispielsweise eine Flavin-, Nicotinamid- oder Pyrrolochinolinchinon-abhängige Oxidoreduktase, umfasst. Vorzugsweise sieht das erfindungsgemäße Verfahren die Verwendung einer Flavin-, Nicotinamid- oder Pyrrolochinolinchinonabhängigen Dehydrogenase vor, wobei Nicotinamid-abhängige Dehydrogenasen, insbesondere NAD(P)/NAD(P)H-abhängige Dehydrogenasen, stärker bevorzugt sind.

Bevorzugt wird als Coenzym-abhängiges Enzym eine Dehydrogenase ausgewählt aus der Gruppe bestehend aus Alkohol-Dehydrogenase (E.C.1.1.1.1; E.C.1.1.1.2), L-Aminosäure-Dehydrogenase (E.C.1.4.1.5), Glucose-Dehydrogenase (E.C. 1.1.1.47), Glucose-6-phosphat-Dehydrogenase (E.C. 1.1.1.49), Glycerin-Dehydrogenase (E.C.1.1.1.6), 3-Hydroxybutyrat-Dehydrogenase (E.C.1.1.1.30), Lactat-Dehydrogenase (E.C.1.1.1.27; E.C.1.1.1.28), Malat-Dehydrogenase (E.C.1.1.1.37) und Sorbitol-Dehydrogenase eingesetzt. Stärker bevorzugt ist das Enzym Glucose-Dehydrogenase (E.C. 1.1.1.47) oder Glucose-6-phosphat-Dehydrogenase (E.C. 1.1.1.49).

Im Rahmen des erfindungsgemäßen Verfahrens können sowohl native als auch mutierte Coenzym-abhängige Enzyme zur Anwendung gelangen. Die Begriffe "mutiertes Coenzym-abhängiges Enzym" bzw. "Mutante", wie sie in vorliegender Anmeldung verwendet werden, bezeichnen dabei jeweils eine genetisch veränderte Variante eines nativen Coenzym-abhängigen Enzyms (Wildtyp-Enzym), welche bei gleicher Anzahl an Aminosäuren eine gegenüber dem Wildtyp-Enzym veränderte Aminosäuresequenz besitzt, d.h. sich in mindestens einer Aminosäure vom Wildtyp-Enzym unterscheidet. Bevorzugt weist die Mutante eine gegenüber dem Wildtyp-Enzym erhöhte thermische oder/und hydrolytische Stabilität auf.

Das mutierte Coenzym-abhängige Enzym kann durch Mutation eines aus einer beliebigen biologischen Quelle stammenden nativen Coenzym-abhängigen Enzyms erhalten werden, wobei der Begriff "biologische Quelle" im Sinne dieser Erfindung sowohl Prokaryoten als auch Eukaryoten umfasst. Die Einführung der Mutation(en) kann ortsspezifisch oder nichtortsspezifisch, bevorzugt ortsspezifisch unter Verwendung von im Fachbereich bekannten rekombinanten Methoden, erfolgen, wobei mindestens ein Aminosäureaustausch innerhalb der Aminosäuresequenz des nativen Enzyms resultiert.

In einer besonders bevorzugten Variante der Erfindung wird als Coenzym-abhängiges Enzym eine mutierte Glucose-Dehydrogenase (E.C. 1.1.1.47) oder eine mutierte Glucose-6-phosphat-Dehydrogenase (E.C.1.1.1.49) verwendet. Beispiele für mutierte Glucose-Dehydrogenasen sind u.a. in WO 2005/045016, WO 2011/020856, Baik (Appl. Environ. Microbiol. (2005), 71, 3285) und Vásquez-Figueroa (ChemBioChem (2007), 8, 2295) beschrieben, auf deren Offenbarung hiermit ausdrücklich Bezug genommen wird. Am stärksten bevorzugt handelt es sich bei dem Coenzym-abhängigen Enzym um eine mutierte Glucose-Dehydrogenase, welche die in SEQ ID NO: 1 (GIucDH_E96G_E17014) oder die in SEQ ID NO: 2 (GlucDH_E170K_K252L) dargestellte Aminosäuresequenz besitzt.

Neben dem Coenzym-abhängigen Enzym umfasst das erfindungsgemäß eingesetzte chemische Nachweisreagenz weiterhin ein artifizielles Coenzym. Ein artifizielles Coenzym im Sinne der vorliegenden Erfindung ist ein gegenüber dem nativen Coenzym chemisch verändertes Coenzym, welches bei Atmosphärendruck eine im Vergleich zum nativen Coenzym höhere Stabilität gegenüber Feuchtigkeit, Temperaturen insbesondere im Bereich von 0°C bis 50°C, Säuren und Basen insbesondere im Bereich von pH 4 bis pH 10, oder/und Nukleophilen wie beispielsweise Alkoholen oder Aminen, aufweist und insofern unter identischen Umgebungsbedingungen über einen längeren Zeitraum als das native Coenzym seine Wirkung entfalten kann.

Vorzugsweise weist das artifizielle Coenzym eine im Vergleich zum nativen Coenzym höhere hydrolytische Stabilität auf, wobei eine vollständige Hydrolysestabilität unter Testbedingungen besonders bevorzugt ist. Im Vergleich zum nativen Coenzym kann das artifizielle Coenzym eine verringerte Bindungskonstante für das Coenzym-abhängige Enzym aufweisen, beispielsweise eine um den Faktor von 2 oder mehr verringerte Bindungskonstante.

Bevorzugte Beispiele für artifizielle Coenzyme, welche im Rahmen des erfindungsgemäßen Verfahrens eingesetzt werden können, sind artifizielle NAD(P)/NAD(P)H-Verbindungen, d.h. chemische Derivate von nativem Nikotinamid-Adenin-Dinukleotid (NAD/NADH) bzw. nativem Nikotinamid-Adenin-Dinukleotidphosphat (NADP/NADPH), oder die Verbindung der Formel (I)

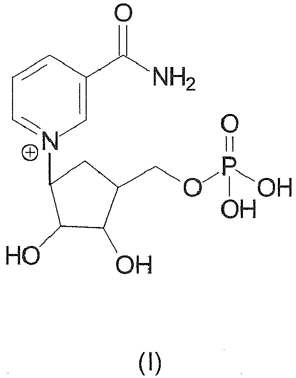

Sofern es sich bei dem artifiziellen Coenzym um eine artifizielle NAD(P)/NAD(P)H-Verbindung handelt, umfasst die artifizielle NAD(P)/NAD(P)H-Verbindung vorzugsweise einen 3-Pyridincarbonyl- oder einen 3-Pyridinthiocarbonyl-Rest, welcher ohne glykosidische Bindung über einen linearen oder cyclischen organischen Rest, insbesondere über einen cyclischen organischen Rest, mit einem phosphorhaltigen Rest, wie beispielsweise einem Phosphatrest, verknüpft ist.

Besonders bevorzugt wird das artifizielle Coenzym aus einer Verbindung der allgemeinen Formel (II) ausgewählt: 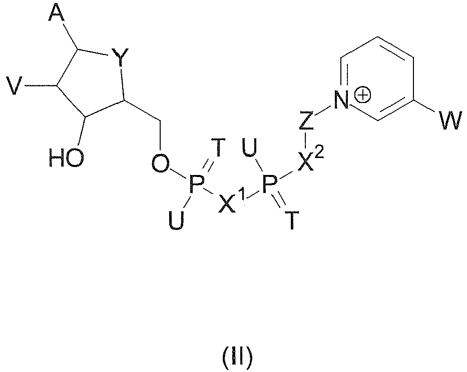 worin
- A =: Adenin oder ein Analogon hiervon,
- T =: jeweils unabhängig O, S,
- U =: jeweils unabhängig OH, SH, BH₃⁻, BCNH₂⁻,
- V =: jeweils unabhängig OH oder eine Phosphatgruppe, oder zwei Gruppen, die eine cyclische Phosphatgruppe bilden;
- W =: COOR, CON(R)₂, COR, CSN(R)₂ mit R = jeweils unabhängig H oder C₁-C₂-Alkyl,
- X¹, X² =: jeweils unabhängig O, CH₂, CHCH₃, C(CH₃)₂, NH, NCH₃,
- Y =: NH, S, O, CH₂,
- Z =: ein linearer oder cyclischer organischer Rest ist, mit der Maßgabe, dass Z und der Pyridin-Rest nicht durch eine glycosidische Verbindung verknüpft sind, oder ein Salz oder eine reduzierte Form davon.

In den Verbindungen der Formel (II) ist Z vorzugsweise ein linearer Rest mit 4-6 C-Atomen, vorzugsweise mit 4 C-Atomen, worin 1 oder 2 C-Atome gegebenenfalls durch ein oder mehrere Heteroatome ausgewählt aus O, S und N ersetzt sind, oder ein Rest umfassend eine cyclische Gruppe mit 5 oder 6 C-Atomen, die gegebenenfalls ein Heteroatom ausgewählt aus O, S und N sowie gegebenenfalls einen oder mehrere Substituenten enthält, und einen Rest CR⁴₂, wobei CR⁴₂ an die cyclische Gruppe und an X² gebunden ist, mit R⁴ = jeweils unabhängig H, F, Cl, CH₃.

Besonders bevorzugt ist Z ein gesättiger oder ungesättigter carbocyclischer oder heterocyclischer Fünfring, insbesondere ein Rest der allgemeinen Formel (III), 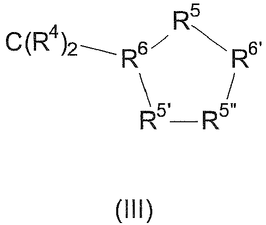 wobei zwischen R⁵' und R⁵" eine Einfach- oder Doppelbindung vorliegen kann, mit
- R⁴ =: jeweils unabhängig H, F, Cl, CH₃,
- R⁵ =: CR⁴₂,
- R⁵'=: O, S, NH, NC₁-C₂-Alkyl, CR⁴₂, CHOH, CHOCH₃, und R⁵" = CR⁴₂, CHOH, CHOCH₃, wenn zwischen R⁵' und R⁵" eine Einfachbindung vorliegt,
- R⁵' = R⁵" = CR⁴,: wenn zwischen R⁵' und R⁵" eine Doppelbindung vorliegt, und
- R⁶, R⁶' =: jeweils unabhängig CH oder CCH₃.

In einer Ausführungsform enthalten die Verbindungen der allgemeinen Formel (II) ein Adenin-Analogon. Der Begriff "Adenin-Analogon", wie er in vorliegender Anmeldung verwendet wird, bezeichnet ein chemisches Derivat von nativem Adenin, welches im menschlichen Körper die gleiche pharmakologische Wirkung entfaltet wie Adenin. Konkrete Beispiele für Adenin-Analoga umfassen insbesondere C₈- und N₆-substituiertes Adenin, 7-Deazaadenin, 8-Azaadenin, 7-Deaza-8-azaadenin und Formycin, wobei die 7-Deazavarianten in der 7-Position mit Halogen, C₁₋₆-Alkinyl, C₁₋₆-Alkenyl oder C₁₋₆-Alkyl substituiert sein können.

In einer weiteren bevorzugten Ausführungsform enthalten die Verbindungen Adenosin-Analoga, welche statt Ribose z.B. 2-Methoxydesoxyribose, 2'-Fluorodesoxyribose, Hexitol, Altritol bzw. polycyclische Analoga, wie Bicyclo-, LNA- und Tricyclo-Zucker enthalten. Insbesondere können in den Verbindungen der Formel (II) auch (Di-)-Phosphatsauerstoffe isotronisch ersetzt sein, wie z.B. O⁻ durch S⁻ bzw. BH₃⁻, O durch NH, NCH₃ bzw. CH₂ und =O durch =S. In den erfindungsgemäßen Verbindungen der Formel (II) ist W vorzugsweise CONH₂ oder COCH₃.

In den Gruppen der Formel (III) ist R⁵ vorzugsweise CH₂. Weiterhin ist bevorzugt, dass R⁵' ausgewählt ist aus CH₂, CHOH und NH. In einer besonders bevorzugten Ausführungsform sind R⁵' und R⁵" jeweils CHOH. In noch einer weiteren bevorzugten Ausführungsform ist R⁵' NH und R⁵" CH₂. Am stärksten bevorzugt ist eine Verbindung der Formel (III), in welcher R⁴ = H ist, R⁵ = CH₂ ist, R⁵' = R⁵" = CHOH ist, und R⁶ = R⁶' = CH ist.

In der am stärksten bevorzugten Ausführungsform handelt es sich bei dem artifiziellen Coenzym um die literaturbekannte Verbindung carbaNAD (J.T. Slama, Biochemistry (1988), 27, 183 und Biochemistry (1989), 28, 7688). Andere stabile Coenzyme, welche erfindungsgemäß Anwendung finden können, sind in WO 98/33936, WO 01/49247, WO 2007/012494, US 5,801,006, US 11/460,366 und der Publikation Blackburn et al. (Chem. Comm. (1996), 2765), beschrieben, auf deren Offenbarung hiermit ausdrücklich Bezug genommen wird.

Sofern gewünscht, kann das chemische Nachweisreagenz neben dem Coenzym-abhängigen Enzym und dem artifiziellen Coenzym weitere Substanzen umfassen, welche dem qualtitativen Nachweis oder/und der quantitativen Bestimmung von Analyten dienen, wie etwa einen Mediator oder/und einen optischen Indikator. Der Begriff "Mediator", wie er in vorliegender Anmeldung verwendet wird, bezeichnet eine chemische Verbindung, welche die Reaktivität des durch Umsetzung mit dem Analyten erhaltenen, reduzierten Coenzyms erhöht und eine Übertragung von Elektronen auf einen geeigneten optischen Indikator oder ein optisches Indikatorsystem ermöglicht. Konkrete Beispiele für Mediatoren umfassen insbesondere Azoverbindungen, Nitrosoaniline, Chinone und Phenazine.

Als optischer Indikator kann erfindungsgemäß eine beliebige Substanz zum Einsatz gelangen, welche reduzierbar ist und bei Reduktion eine visuell oder/und maschinell detektierbare Änderung ihrer optischen Eigenschaften, wie beispielsweise Farbe, Fluoreszenz, Remission, Transmission, Polarisation oder/und Brechungsindex, erfährt. Bevorzugte optische Indikatoren im Sinne der vorliegenden Erfindung umfassen reduzierbare Heteropolysäuren, insbesondere 2,18-Phosphormolybdänsäure. Alternativ können auch Chinone, wie beispielsweise Resazurin, Dichlorphenolindophenol oder/und Tetrazoliumsalze, als optische Indikatoren eingesetzt werden.

Nach Bereitstellen einer Charge des chemischen Nachweisreagenzes, welche vorzugsweise in flüssiger Form und besonders bevorzugt als Suspension vorliegt, wird ein erster Teil dieser Charge auf einen ersten Träger, wie beispielsweise eine Folie oder ein Spritzgussteil, aufgebracht und anschließend getrocknet, wodurch eine Beschichtung des ersten Trägers mit der ersten Charge des chemischen Nachweisreagenzes erfolgt. Während sowohl dieser Schritt als auch nachfolgende Verarbeitungsschritte bei Verwendung konventioneller Testchemie, welche gegenüber Feuchtigkeit, Wärme oder/und Licht empfindlich ist, nur kurze Zeit in Anspruch nehmen dürfen, kann durch den Einsatz einer Kombination aus Coenzym-abhängigem Enzym und artifiziellem Coenzym und die hierdurch erzielte Langzeitstabilität der Testchemie auf eine sofortige Weiterverarbeitung der kompletten Charge des chemischen Nachweisreagenzes verzichtet werden, was erhebliche produktionstechnische Vorteile mit sich bringt.

Infolge der Unempfindlichkeit des erfindungsgemäß verwendeten chemischen Nachweisreagenzes gegenüber Feuchtigkeit, Wärme und Licht lassen sich deutlich größere Chargen des chemischen Nachweisreagenzes erzeugen, ohne dass während des Produktions- bzw. Verarbeitungsprozesses umweltbedingte Verschlechterungen der Testchemie eintreten. Dementsprechend kann mit einer einzelnen Charge des chemischen Nachweisreagenzes auch eine größere Anzahl an Trägern beschichtet werden, womit letztlich die Erzeugung einer größeren Charge an diagnostischen Elementen ermöglicht wird, welche zudem homogener ist als eine einzelne Charge diagnostischer Elemente, welche konventionelle Testchemie umfasst.

Darüber hinaus hat das oben beschriebene chemische Nachweisreagenz den Vorteil, dass mittels einer einzelnen Charge mehrere Chargen an diagnostischen Elementen erzeugt werden können, welche alsdann untereinander homogen sind. Auf diese Weise wird sichergestellt, dass selbst bei einer großen Charge des chemischen Nachweisreagenzes die ersten hieraus hergestellten diagnostischen Elemente und die zuletzt hergestellten diagnostischen Elementen identisch sind oder lediglich innerhalb enger Grenzen variieren. Demgegenüber müssen bei der Verwendung konventioneller Testchemie kleine Chargen an diagnostischen Testelemeneten produziert werden, damit sich die Streuung der sich durch Umwelteinflüsse verschlechternden Reaktivität des chemischen Nachweisreagenzes noch innerhalb des zulässigen Rahmens bewegt.

Im Anschluss an die Beschichtung wird der beschichtete erste Träger mittels geeigneter Techniken zu mehreren ersten diagnostischen Elementen vereinzelt (Vorbatch) und anhand mindestens eines der ersten diagnostischen Elemente eine Chargenkodierung für die komplette Charge des chemischen Nachweisreagenzes erstellt. Die in diesem Zusammenhang erstellte Chargenkodierung enthält vorzugsweise eine mathematische Gleichung, welche die gegebenenfalls temperaturabhängige Beziehung zwischen der jeweiligen Menge eines zu bestimmenden Analyten und dem hieraus resultierenden, beispielsweise optisch oder elektrochemisch messbaren Signal angibt.

Als Chargenkodierung kann grundsätzlich jeder beliebige Code verwendet werden, welcher dem Fachmann für die Kodierung diagnostischer Elemente angemessen erscheint und unter Einsatz geeigneter Mittel eine zuverlässige Rückverfolgung der mit dem Code versehenen diagnostischen Elemente erlaubt. Bevorzugt wird als Chargenkodierung ein optisch oder/und elektronisch auslesbarer Code verwendet, wie beispielsweise ein Barcode oder ein RFID-Transponder. Besonders bevorzugt gelangt im Rahmen des erfindungsgemäßen Verfahrens ein Barcode zur Anwendung, welcher ein- oder zweidimensional bereitgestellt sein kann, in Schwarzweiß, in Graustufen oder in Farbe ausgestaltet sein kann und, sofern gewünscht, ein Hologramm umfassen kann. Alternativ besteht speziell im Falle der Herstellung elektrochemisch zu detektierender Testelemente die Möglichkeit, eine Kodierung über eine elektrische Kontaktierung der einzelnen diagnostischen Elemente zu realisieren.

Nach der Erstellung der Chargenkodierung sieht das erfindungsgemäße Verfahren die Beschichtung eines zweiten Trägers, insbesondere einer Folie oder eines Spritzgussteils, mit einem zweiten Teil der Charge des chemischen Nachweisreagenzes vor. Nach Aufbringen des chemischen Nachweisreagenzes auf den zweiten Träger und nachfolgende Trocknung wird ein beschichteter zweiter Träger erhalten, aus welchem durch anschließende Vereinzelung mehrere zweite diagnostische Elemente erhalten werden, die bei Bedarf einer Überprüfung unterzogen und nach Bestehen der Qualitätskontrolle in geeigneter Weise verpackt werden können.

Die Kodierung der zweiten diagnostischen Elemente erfolgt erfindungsgemäß vor dem Vereinzeln des beschichteten zweiten Trägers. Hierbei werden die zweiten diagnostischen Elemente mit der anhand des Vorbatches (d.h. mit der anhand der ersten diagnostischen Elemente) erstellten Chargenkodierung versehen, was vorzugsweise durch Aufbringen der Chargenkodierung auf den zweiten Träger vor dem Beschichten des zweiten Trägers mit dem zweiten Teil der Charge des chemischen Nachweisreagenzes realisiert wird. Dies hat erhebliche finanzielle und produktionstechnische Vorteile, da eine einzige Chargenkodierung ausreicht, um eine um den Faktor 2, bevorzugt um den Faktor 3, besonders bevorzugt um den Faktor 5 erhöhte Menge an diagnostischen Elementen zu kodieren wie bei Einsatz konventioneller Testchemie.

Gleichzeitig ermöglicht das erfindungsgemäße Verfahren durch gezieltes Aufbringen der Chargenkodierung auf den zweiten Träger eine Kodierung jedes einzelnen diagnostischen Elements, was bei Verwendung konventioneller Testchemie im Allgemeinen nicht möglich ist. Wie vorstehend erläutert, erfordert die Erstellung einer Chargenkodierung üblicherweise die Herstellung eines Vorbatches. Die anschließende Kodierung diagnostischer Elemente mit der hierbei erhaltenen Chargenkodierung muss jedoch in einem früheren Produktionsschritt, und in jedem Fall vor der Vereinzelung des jeweiligen Trägers zu den diagnostischen Elementen, erfolgen, um wirtschaftlich vertretbar und technisch machbar zu sein.

Allerdings erfordert die Erstellung einer Chargenkodierung anhand eines Vorbatches auch eine Unterbrechung der Produktion. Mit konventioneller Testchemie, welche eine geringe Stabilität gegenüber Feuchtigkeit, Wärme oder/und Licht aufweist, ist eine solche Unterbrechung der Produktion zur Erstellung einer Chargenkodierung indessen nicht möglich, da bei einer längeren Lagerzeit bzw. einem länger laufenden Verarbeitungsprozess Verschlechterungen der Testchemie eintreten. Die Chargenkodierung eines solchen Vorbatches würde sich folglich stark und nicht-reproduzierbar von der Chargenkodierung einer nachfolgend produzierten Charge diagnostischer Elemente unterscheiden.

Durch die erfindungsgemäß realisierbare Kodierung einzelner diagnostischer Elemente wird die Verwendung von ROM-Keys oder die Eingabe von Codenummern durch den Verbraucher verzichtbar. Der Vorteil der Kodierung einzelner diagnostischer Elemente gegenüber der Kodierung eines ganzen Magazins (z.B. einer Dose mit Teststreifen) ist vor allem bei Elementen zu sehen, bei denen der Verbraucher die Kodierung selbstständig vornehmen muss, dies aber aus Bequemlichkeit, Vergesslichkeit oder Unwissenheit unterlässt. Durch die Kodierung einzelner diagnostischer Elemente wird somit das Risiko, dass der Verbraucher eine Messung mit einem unpassenden Code (z.B. mit einer früheren Charge des diagnostischen Elements) durchführt, ausgeschlossen.

Infolge der Unempfindlichkeit des erfindungsgemäß verwendeten chemischen Nachweisreagenzes gegenüber Feuchtigkeit, Wärme und Licht muss eine Charge des chemischen Nachweisreagenzes nicht unmittelbar nach ihrer Herstellung weiterverarbeitet werden, um eine Änderung ihrer chemischen oder/und physikalischen Eigenschaften zu vermeiden. Vielmehr ermöglicht das erfindungsgemäße Verfahren, dass zwischen der erstmaligen Bereitstellung einer Charge des chemischen Nachweisreagenzes und ihrer vollständigen Verarbeitung zu diagnostischen Elementen ein Zeitraum von mehreren Stunden bis hin zu mehreren Wochen liegen kann, was sowohl zeitliche als auch produktionstechnische Vorteile bietet.

In einer bevorzugten Variante des erfindungsgemäßen Verfahrens liegt zwischen der Bereitstellung der Charge des chemischen Nachweisreagenzes und der im Anschluss an die Erstellung der Chargenkodierung erfolgenden Beschichtung des zweiten Trägers mit einem zweiten Teil der Charge des chemischen Nachweisreagenzes ein Zeitraum von mindestens 36 Stunden, bevorzugt von mindestens 48 Stunden. Darüber hinaus ist es aus produktionstechnischen Gründen bevorzugt, dass zwischen der Beschichtung des zweiten Trägers mit dem zweiten Teil der Charge des chemischen Nachweisreagenzes und der Vereinzelung des beschichteten zweiten Trägers zu mehreren zweiten diagnostischen Elementen ein Zeitraum von mindestens 20 Tagen, bevorzugt von mindestens 30 Tagen liegt.

Im einzelnen wird der Zeitraum zwischen der Bereitstellung der Charge des chemischen Nachweisreagenzes und der Beschichtung des zweiten Trägers, oder/und zwischen der Beschichtung des zweiten Trägers und dessen Vereinzelung zu mehreren zweiten diagnostischen Elementen vorzugsweise derart gewählt, dass die Charge des chemischen Nachweisreagenzes im wesentlichen keine Änderung ihrer chemischen oder/und physikalischen Eigenschaften erfährt und dementsprechend die Herstellung diagnostischer Elemente ermöglicht, welche eine Bestimmung von Analyten innerhalb des gesetzlich zulässigen Rahmens sicherstellen.

Der Ausdruck "im wesentlichen keine Änderung der chemischen oder/und physikalischen Eigenschaften" bedeutet in diesem Zusammenhang eine Abnahme der Aktivität des Coenzym-abhängigen Enzyms bzw. eine Abnahme des Gehalts an artifiziellem Coenzym in dem chemischen Nachweisreagenz von weniger als 40%, bevorzugt von weniger als 30%, und am stärksten bevorzugt von weniger als 20%, bezogen auf die Aktivität des Coenzym-abhängigen Enzyms bzw. bezogen auf den Gehalt an artifiziellem Coenzym in dem chemischen Nachweisreagenz unmittelbar nach dessen Herstellung.

Die mittels des erfindungsgemäßen Verfahrens erzeugten ersten diagnostischen Elemente und zweiten diagnostischen Elemente können gleich oder unterschiedlich ausgebildet sein, sind jedoch bevorzugt gleich ausgebildet. Die diagnostischen Elemente können prinzipiell jede dem Fachmann geläufige physikalische Form aufweisen, welche für die Bestimmung des Vorhandenseins oder/und der Menge eines Analyten in einer Probe geeignet ist, und umfassen jeweils mindestens ein Testfeld, welches mit einer den Analyten enthaltenden Probe in Kontakt gebracht werden kann und unter Verwendung geeigneter Mittel eine qualitative oder/und quantitative Bestimmung des Analyten ermöglicht.

Bevorzugte Beispiele für erste diagnostische Elemente oder/und zweite diagnostische Elemente umfassen insbesondere Teststreifen, Testbänder und Testdiscs, aus denen bei Bedarf hierauf basierende diagnostische Elemente, wie beispielsweise Teststreifenmagazine und Bandmagazine, hergestellt werden können. Geeignete Teststreifenmagazine umfassen insbesondere Blistermagazine, Leporellomagazine, Scheibenmagazine, Stapelmagazine, Trommelmagazine und Wendemagazine, welche u.a. in EP 0 951 939, EP 1 022 565, EP 1 736 772, WO 2005/104948 und WO 2010/094427 beschrieben sind. Bandmagazine sind u.a. aus DE 10 2005 013 685, EP 1 739 432 und WO 2004/047642 bekannt. Auf die Offenbarung vorstehender Druckschriften wird hiermit ausdrücklich Bezug genommen.

Bevorzugt umfassen die ersten diagnostischen Elemente oder/und die zweiten diagnostischen Elemente jeweils mehrere Testfelder, bevorzugt mindestens 10 einzelne Testfelder, stärker bevorzugt mindestens 25 einzelne Testfelder, und am stärksten bevorzugt mindestens 50 einzelne Testfelder. Vorzugsweise sind die einzelnen Testfelder hierbei jeweils in einem Abstand von wenigen Millimetern bis zu wenigen Zentimetern, beispielsweise in einem Abstand von < 2.5 cm, voneinander angeordnet.

Besonders bevorzugt ist jedes der Testfelder der ersten diagnostischen Elemente oder/und der zweiten diagnostischen Elemente zumindest teilweise von einem hydrophoben Rand umgeben oder/und ist in einer eigenen, im wesentlichen geschlossenen Kammer gelagert. Die Verwendung eines hydrophoben Rands ist insbesondere in Fällen von Vorteil, in welchen ein Verbraucher eine Probe des zu bestimmenden Analyten manuell auf ein Testfeld des jeweiligen diagnostischen Elements aufbringen muss und ein Überlaufen der Probe auf benachbarte Testfelder oder ein Eintritt der Probe in ein zur Vermessung des diagnostischen Elements benötigtes Gerät vermieden werden soll.

Diagnostische Elemente, in welchen jedes Testfeld in einer eigenen, im wesentlichen geschlossenen Kammer gelagert ist, finden beispielsweise bei vollautomatischen Messsystemen Anwendung. Derartige Messsysteme weisen mehrere Testfelder, die z.B. ringförmig nebeneinander auf einer drehbaren Platte angeordnet sein können, und ebenso viele, zum Anritzen von Haut ausgebildete Nadelelemente in magazinierter Form auf. Obwohl in diesen Fällen keine manuelle Aufbringung der Probe auf ein Testfeld seitens des Verbrauchers vorgenommen werden muss (dies erledigt das System automatisch), müssen sich die einzelnen Testfelder aus hygienischen Gründen in voneinander getrennten Kammern befinden. Der Ausdruck "im wesentlichen geschlossene Kammer" bedeutet in diesem Zusammenhang, dass die Kammerwände für Luft oder/und Wasser durchlässig sein können, jedoch keinen Eintritt von Staubpartikeln in die Kammer ermöglichen und somit eine staubfreie Lagerung der Testfelder gewährleisten.

Enthalten die Testfelder eines diagnostischen Elements konventionelle Testchemie, welche eine geringe Stabilität gegenüber Feuchtigkeit, Wärme oder/und Licht aufweist, so muss der die Testfelder zumindest teilweise umgebende hydrophobe Rand bzw. müssen die die Testfelder umschließenden Kammern in einem komplizierten und technisch aufwändigen Verfahren hergestellt werden. Aufgrund der Tatsache, dass das erfindungsgemäß eingesetzte chemischen Nachweisreagenz u.a. gegenüber Licht, und insbesondere gegenüber UV-Licht, stabil ist, können die in vorliegender Anmeldung beschriebenen diagnostischen Elemente indessen auch lichthärtbare bzw. UV-härtbare Materialien umfassen. Insofern sieht das erfindungsgemäße Verfahren in einer bevorzugten Variante vor, dass der die Testfelder eines diagnostischen Elements zumindest teilweise umgebende hydrophobe Rand oder/und die Kammerwände der die einzelnen Testfelder umschließenden Kammern aus einem UV-härtbaren Material gebildet sind. Konkrete Beispiele für UV-härtbare Materialien, welche in diesem Zusammenhang Anwendung finden können, sind dem Fachmann bekannt und umfassen u.a. Epoxid- und Acrylat-Klebstoffe, sind jedoch nicht auf diese beschränkt. Darüber hinaus ist es erfindungsgemäß möglich, Licht gezielt zur Generierung einer Schicht des chemischen Nachweisreagenzes auf den diagnostischen Elementen zu nutzen.

Aufgrund der Stabilität des erfindungsgemäß eingesetzten chemischen Nachweisreagenzes gegenüber Licht ergeben sich darüber hinaus signifikante Vorteile in Bezug auf die Produktion sowie eine eventuelle Qualitätskontrolle hieraus hergestellter diagnostischer Elemente. So müssen zum einen keinerlei produktionstechnisch aufwändige Maßnahmen ergriffen werden, welche einem Schutz der diagnostischen Elemente vor Licht dienen. Auf diese Weise ist es möglich, die Produktion erheblich zu vereinfachen und gleichzeitig die Produktionskosten zu verringern.

Andererseits können diagnostische Elemente, welche mit einem erfindungsgemäß eingesetzten chemischen Nachweisreagenz beschichtet sind, beispielsweise mittels optischer Methoden auf mögliche Defekte oder auf eine ungleichmäßige Verteilung der Beschichtung untersucht werden. Dementsprechend umfasst das erfindungsgemäße Verfahren in einer bevorzugten Variante zusätzlich eine Überprüfung der Dicke oder/und der Homogenität der Schicht des chemischen Nachweisreagenzes auf den zweiten diagnostischen Elementen.

Gleichzeitig weisen diagnostische Elemente, welche mit einem erfindungsgemäß eingesetzten chemischen Nachweisreagenz beschichtet sind, auch eine hohe Stabilität gegenüber Feuchtigkeit auf. Demzufolge werden diagnostische Elemente, welche mittels des erfindunsgemäßen Verfahrens hergestellt und anschließend verpackt worden sind, vorzugsweise keiner Wasserdampfdichtigkeitsprüfung unterzogen, wodurch der gesamte Fertigungsprozess beschleunigt und die Produktionskosten verringert werden können.

Die mittels des erfindungsgemäßen Verfahrens hergestellten diagnostischen Elemente können zur qualitativen oder/und quantitativen Bestimmung einer beliebigen biologischen oder chemischen Substanz verwendet werden, welche optisch oder elektrochemisch nachweisbar ist. Vorzugsweise wird der Analyt aus der Gruppe bestehend aus Äpfelsäure, Alkohol, Ascorbinsäure, Cholesterin, Glucose, Glycerin, Harnstoff, 3-Hydroxybutyrat, Milchsäure, Pyruvat und Triglyceriden ausgewählt, wobei Glucose besonders bevorzugt ist.

Der zu bestimmende Analyt kann aus einer beliebigen Quelle stammen, ist jedoch bevorzugt in einem Körperfluid, umfassend, jedoch nicht beschränkt auf, Vollblut, Plasma, Serum, Lymphflüssigkeit, Gallenflüssigkeit, Cerebrospinalflüssigkeit, extrazelluläre Gewebeflüssigkeit, Harn, sowie Drüsensekrete wie beispielsweise Speichel oder Schweiß, enthalten. Bevorzugt wird mittels des erfindungsgemäßen Nachweisreagenzes indessen das Vorhandensein oder/und die Menge eines Analyten in einer Probe ausgewählt aus der Gruppe bestehend aus Vollblut, Plasma und Serum bestimmt.

In einem weiteren Aspekt betrifft die Erfindung ein diagnostisches Produkt, welches (a) mindestens ein diagnostisches Element, und (b) ggf. mindestens ein Nadelelement zum Anritzen von Haut umfasst. Das diagnostische Element umfasst seinerseits (i) einen Träger, (ii) ein chemisches Nachweisreagenz, umfassend ein Coenzym-abhängiges Enzym und ein artifizielles Coenzym, welches auf den Träger in Form einer Schicht aufgebracht ist, und (iii) eine Chargenkodierung.

In einer bevorzugten Variante umfasst das erfindungsgemäße diagnostische Produkt neben dem chemischen Nachweisreagenz weiterhin ein Nadelelement zum Anritzen von Haut, welches vorzugsweise aus einem sterilisierbaren Material, wie beispielsweise aus Metall oder Kunststoff, besteht. Um einen Transfer des zu untersuchenden Körperfluids, wie beispielsweise von Blut, auf das diagnostische Element zu ermöglichen, umfasst das Nadelelement vorzugsweise einen Kapillarkanal, mittels welchem eine zur Bestimmung des Analyten ausreichende Menge der Probe aufgenommen und unter Ausnutzung von Kapillarkräften auf ein Testfeld des diagnostischen Elements aufgebracht werden kann.

Was bevorzugte Ausführungsformen des diagnostischen Elements (inklusive der bevorzugten Varianten für Träger, chemisches Nachweisreagenz und Chargenkodierung) betrifft, so wird auf die Ausführungen in Zusammenhang mit der Beschreibung des erfindungsgemäßen Verfahrens verwiesen.

In noch einem weiteren Aspekt betrifft die Erfindung die Verwendung eines chemischen Nachweisreagenzes, umfassend ein Coenzym-abhängiges Enzym und ein artifizielles Coenzym, zur Erhöhung der Chargengröße oder/und Chargenhomogenität bei der Herstellung von diagnostischen Elementen, wobei die einzelnen diagnostischen Elemente einer Charge jeweils im wesentlichen identisch sind.

In einer bevorzugten Variante kann durch Verwendung des oben beschriebenen chemischen Nachweisreagenzes die Chargengröße gegenüber einem chemischen Nachweisreagenz, welches ein Coenzym-abhängiges Enzym und ein natives Coenzym umfasst, um den Faktor 2, bevorzugt um den Faktor 3, besonders bevorzugt um den Faktor 5 erhöht werden.

Was bevorzugte Ausführungsformen des chemischen Nachweisreagenzes sowie der diagnostischen Elemente betrifft, so wird wiederum auf die Ausführungen in Zusammenhang mit der Beschreibung des erfindungsgemäßen Verfahrens verwiesen.

Die Erfindung soll durch die nachfolgenden Figuren und Beispiele näher erläutert werden:

### Beschreibung der Figuren

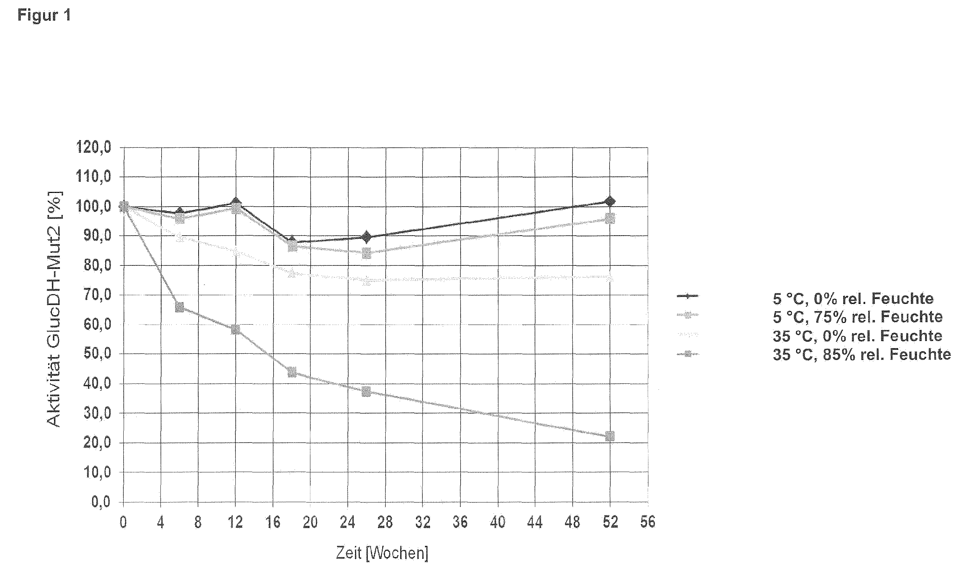

**Figur 1**: Darstellung der Aktivität von Glucose-Dehydrogenase-Doppelmutante GlucDH_E96G_E170K (GlucDH-Mut2) bei Lagerung des Enzyms in Gegenwart von carbaNAD über einen Zeitraum von 52 Wochen bei einer Temperatur von 5°C bzw. 35°C und einer relativen Luftfeuchtigkeit von 0% (Trockenmittel), 75% bzw. 85%.

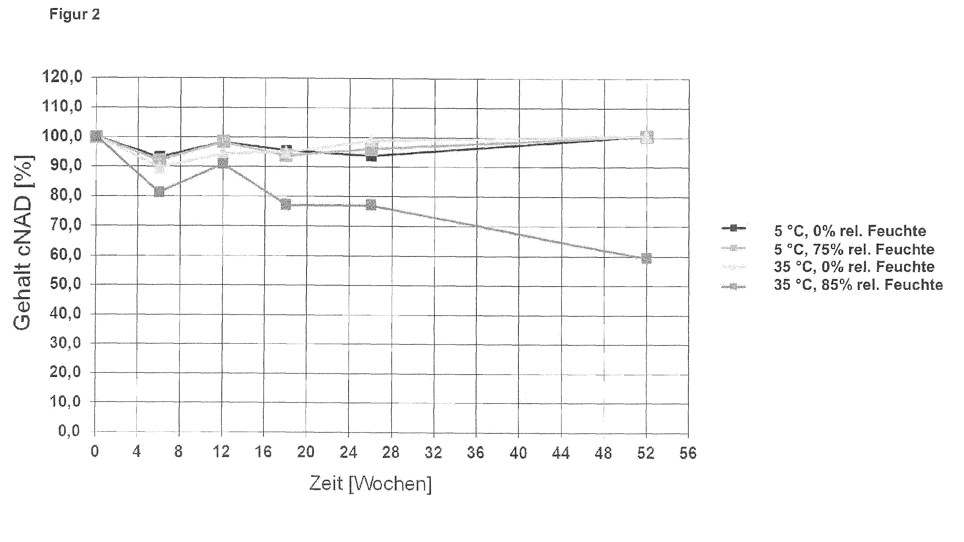

**Figur 2****:** Darstellung des Gehalts an carbaNAD (cNAD) bei Lagerung des Coenzyms in Gegenwart von Glucose-Dehydrogenase-Doppelmutante GlucDH_E96G_E170K über einen Zeitraum von 52 Wochen bei einer Temperatur von 5°C bzw. 35°C und einer relativen Luftfeuchtigkeit von 0% (Trockenmittel), 75% bzw. 85%.

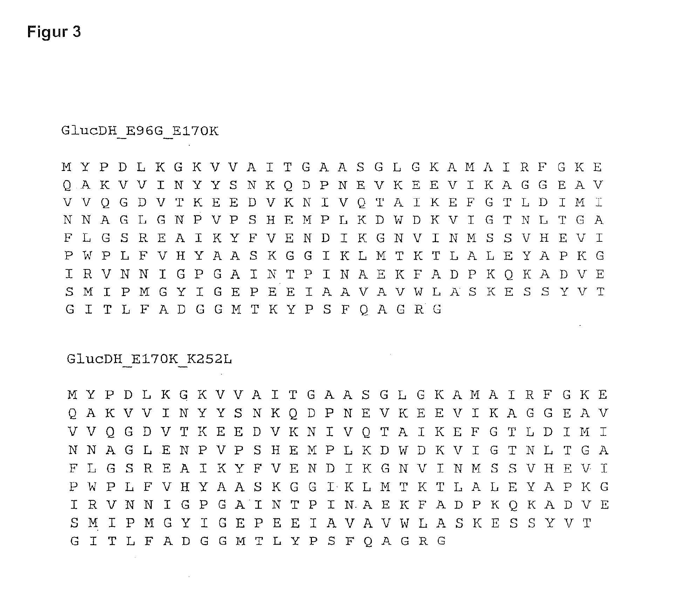

**Figur 3****:** Darstellung der Aminosäuresequenzen der Glucose-Dehydrogenase-Doppelmutanten GlucDH_E96G_E170K (GlucDH-Mut1) und GlucDH_E170K_K252L (GlucDH-Mut2), welche durch Mutation von Wildtyp-Glucose-Dehydrogenase aus Bacillus subtilis erhalten wurden.

### Beispiel 1

Ein Gemisch aus Glucose-Dehydrogenase-Doppelmutante GlucDH_E170K_K252L und carbaNAD wurde (a) bei einer Temperatur von 5°C und einer relativen Luftfeuchtigkeit von 0% (d.h. in Gegenwart eines Trockenmittels), (b) bei einer Temperatur von 5°C und einer relativen Luftfeuchtigkeit von 75%, (c) bei einer Temperatur von 35°C und einer relativen Luftfeuchtigkeit von 0%, bzw. (d) bei einer Temperatur von 35°C und einer relativen Luftfeuchtigkeit von 85% über einen Zeitraum von jeweils 52 Wochen eingelagert.

Anschließend wurde in regelmäßigen Abständen die Aktivität der Glucose-Dehydrogenase-Doppelmutante GlucDH_E170K_K252L sowie der Gehalt an carbaNAD in dem Gemisch aus Enzym und artifiziellem Coenzym bestimmt. Eine graphische Darstellung der Ergebnisse dieser Bestimmungen ist den Figuren 1 und 2 der vorliegenden Anmeldung zu entnehmen.

Im Rahmen dieser Messungen zeigte sich, dass die Aktivität des Enzyms nach Lagerung für 52 Wochen bei einer Temperatur von 5°C und einer relativen Luftfeuchtigkeit von 0% bei knapp 100% liegt. Nach Einlagerung des Gemisches bei einer Temperatur von 5°C und einer relativen Luftfeuchtigkeit von 75% wird noch eine Enzymaktivität von etwa 95%, bezogen auf den Ausgangswert, gemessen (siehe Figur 1).

Wird das Enzym für 52 Wochen bei einer Temperatur von 35°C und einer relativen Luftfeuchtigkeit von 0% eingelagert, beträgt die Restaktivität noch etwa 75%. Nach Einlagerung des Gemisches aus Enzym und artifiziellem Coenzym bei einer Temperatur von 35°C und einer relativen Luftfeuchtigkeit von 85% wird nach 52 Wochen eine Enzymaktivität von etwa 20%, bezogen auf den Ausgangswert, beobachtet.

Was die Stabilität von carbaNAD in dem Gemisch aus Enzym und artifiziellem Coenzym betrifft, so geht aus Figur 2 hervor, dass der Gehalt an carbaNAD nach Lagerung für 52 Wochen bei einer Temperatur von 5°C sowohl bei einer relativen Luftfeuchtigkeit von 0% als auch bei einer relativen Luftfeuchtigkeit von 75% bei knapp 100%, bezogen auf den Ausgangswert, liegt.

Selbst bei Einlagerung des Gemisches aus Enzym und artifiziellem Coenzym für 52 Wochen bei einer Temperatur von 35°C und einer relativen Luftfeuchtigkeit von 0% beträgt der Gehalt an carbaNAD nach dieser Zeit etwa 100%. Wird das Gemisch bei einer Temperatur von 35°C und einer relativen Luftfeuchtigkeit von 85% eingelagert, so ergibt sich nach 52 Wochen noch ein Coenzymgehalt von etwa 60%, bezogen auf den Ausgangswert.

Anhand dieser Daten zeigt sich, dass carbaNAD bei erhöhten Temperaturen oder/und bei erhöhter relativer Luftfeuchtigkeit über einen langen Zeitraum stabil ist. Gleichzeitig wird deutlich, dass die Glucose-Dehydrogenase-Doppelmutante GlucDH_E170K_K252L in Gegenwart von carbaNAD eine hohe Stabilität aufweist, was insbesondere Vorteile bei der Herstellung und Verarbeitung von diagnostischen Elementen für die Glucosebestimmung mit sich bringt.

## Patentansprüche

1. Verfahren zur Herstellung von diagnostischen Elementen, umfassend die Schritte:
(a) Bereitstellen einer Charge eines chemischen Nachweisreagenzes, umfassend ein Coenzym-abhängiges Enzym und ein artifizielles Coenzym,
(b) Beschichten eines ersten Trägers mit einem ersten Teil der Charge des chemischen Nachweisreagenzes,
(c) Vereinzeln des beschichteten ersten Trägers zu mehreren ersten diagnostischen Elementen,
(d) Erstellen einer Chargenkodierung für die Charge des chemischen Nachweisreagenzes anhand mindestens eines der ersten diagnostischen Elemente,
(e) Beschichten eines zweiten Trägers mit einem zweiten Teil der Charge des chemischen Nachweisreagenzes,
(f) Vereinzeln des beschichteten zweiten Trägers zu mehreren zweiten diagnostischen Elementen, und
(g) ggf. Überprüfen und Verpacken der zweiten diagnostischen Elemente,
**dadurch gekennzeichnet,**
**dass** die zweiten diagnostischen Elemente vor dem Vereinzeln des beschichteten zweiten Trägers mit der in Schritt (d) erstellten Chargenkodierung versehen werden.

2. Verfahren nach Anspruch 1,
**dadurch gekennzeichnet,**
**dass** zwischen den Schritten (a) und (e) ein Zeitraum von mindestens 36 Stunden, bevorzugt von mindestens 48 Stunden liegt oder/und zwischen den Schritten (e) und (f) ein Zeitraum von mindestens 20 Tagen, bevorzugt von mindestens 30 Tagen liegt.

3. Verfahren nach Anspruch 1 oder 2,
**dadurch gekennzeichnet,**
**dass** die Charge des chemischen Nachweisreagenzes zwischen den Schritten (a) und (e) oder/und zwischen den Schritten (e) und (f) im wesentlichen keine Änderung ihrer chemischen oder/und physikalischen Eigenschaften erfährt.

4. Verfahren nach einem der Ansprüche 1 bis 3,
**dadurch gekennzeichnet,**
**dass** die Charge des chemischen Nachweisreagenzes in flüssiger Form, insbesondere als Suspension, vorliegt.

5. Verfahren nach einem der Ansprüche 1 bis 4,
**dadurch gekennzeichnet,**
**dass** als Coenzym-abhängiges Enyzm eine NAD(P)/NAD(P)H-abhängige Dehydrogenase, insbesondere eine mutierte NAD(P)/NAD(P)H-abhängige Glucose-Dehydrogenase (EC 1.1.1.47) oder Glucose-6-phosphat-Dehydrogenase (EC 1.1.1.49), verwendet wird.

6. Verfahren nach einem der Ansprüche 1 bis 5,
**dadurch gekennzeichnet,**
**dass** als artifizielles Coenzym eine artifizielle NAD(P)/NAD(P)H-Verbindung, insbesondere carba-NAD, oder die Verbindung der Formel (I) 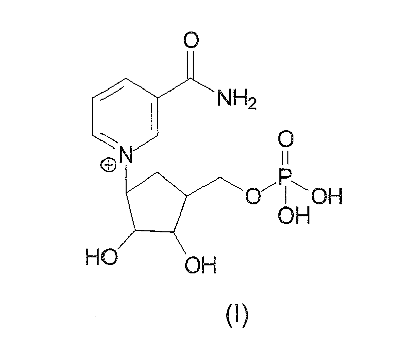 verwendet wird.

7. Verfahren nach einem der Ansprüche 1 bis 6,
**dadurch gekennzeichnet,**
**dass** der zweite Träger vor dem Beschichten mit dem zweiten Teil der Charge des chemischen Nachweisreagenzes mit der Chargenkodierung versehen wird.

8. Verfahren nach einem der Ansprüche 1 bis 7,
**dadurch gekennzeichnet,**
**dass** als Chargenkodierung ein optisch oder/und ein elektronisch auslesbarer Code, insbesondere ein Barcode, verwendet wird.

9. Verfahren nach einem der Ansprüche 1 bis 8,
**dadurch gekennzeichnet,**
**dass** die ersten diagnostischen Elemente oder/und die zweiten diagnostischen Elemente jeweils mehrere Testfelder umfassen.

10. Verfahren nach Anspruch 9,
**dadurch gekennzeichnet,**
**dass** jedes Testfeld zumindest teilweise von einem hydrophoben Rand umgeben ist oder/und in einer eigenen, im wesentlichen geschlossenen Kammer gelagert ist.

11. Verfahren nach Anspruch 10,
**dadurch gekennzeichnet,**
**dass** der hydrophobe Rand oder/und die Kammerwände aus einem UV-härtbaren Material gebildet sind.

12. Verfahren nach einem der Ansprüche 1 bis 11,
**dadurch gekennzeichnet,**
**dass** in Schritt (g) die Dicke oder/und die Homogenität der Schicht des chemischen Nachweisreagenzes überprüft wird.

13. Verfahren nach einem der Ansprüche 1 bis 12,
**dadurch gekennzeichnet,**
**dass** es keine Überprüfung der Wasserdampfdichtigkeit der verpackten diagnostischen Elemente umfasst.

14. Diagnostisches Produkt, umfassend:
(a) mindestens ein diagnostisches Element, umfassend
(i) einen Träger,
(ii) ein chemisches Nachweisreagenz, umfassend ein Coenzym-abhängiges Enzym und ein artifizielles Coenzym, welches auf den Träger in Form einer Schicht aufgebracht ist, und
(iii) eine Chargenkodierung, und
(b) ggf. mindestens ein Nadelelement zum Anritzen von Haut.

15. Diagnostisches Produkt nach Anspruch 14,
**dadurch gekennzeichnet,**
**dass** das diagnostische Element mehrere Testfelder umfasst.

16. Diagnostisches Produkt nach Anspruch 15,
**dadurch gekennzeichnet,**
**dass** jedes Testfefd zumindest teilweise von einem hydrophoben Rand umgeben ist oder/und in einer eigenen, im wesentlichen geschlossenen Kammer gelagert ist.

17. Diagnostisches Produkt nach Anspruch 16,
**dadurch gekennzeichnet,**
**dass** der hydrophobe Rand oder/und Kammerwände aus einem UV-härtbaren Material gebildet sind.

18. Diagnostisches Produkt nach einem der Ansprüche 15 bis 17,
**dadurch gekennzeichnet,**
**dass** das diagnostische Element als Teststreifen, Testband, Testdisc,
Bandmagazin oder Teststreifenmagazin ausgebildet ist.

19. Verwendung eines chemischen Nachweisreagenzes, umfassend ein Coenzym-abhängiges Enzym und ein artifizielles Coenzym, zur Erhöhung der Chargengröße oder/und Chargenhomogenität bei der Herstellung von diagnostischen Elementen, wobei die einzelnen diagnostischen Elemente einer Charge jeweils im wesentlichen identisch sind.

20. Verwendung nach Anspruch 19, wobei die Chargengröße gegenüber einem chemischen Nachweisreagenz, welches ein Coenzym-abhängiges Enzym und ein natives Coenzym umfasst, um den Faktor 2, bevorzugt um den Faktor 3, besonders bevorzugt um den Faktor 5 erhöht ist.
